# EUROPÄISCHE PATENTSCHRIFT

(11) **EP 1 051 251 B1**
(45) Veröffentlichungstag und Bekanntmachung des Hinweises auf die Patenterteilung: **26.02.2003**
(21) Anmeldenummer: 98955548.7
(22) Anmeldetag: 06.11.1998
(51) Int. Cl.: B01J 23/56, B01J 23/44, C07C 19/08, C07C 17/23

(54) **VERFAHREN ZUR HERSTELLUNG VON FLUORKOHLENWASSERSTOFFEN**
METHOD FOR PRODUCING FLUOROCARBONS
PROCEDE PERMETTANT DE PRODUIRE DES FLUOROCARBONES

(30) Priorität: 06.11.1997 DE 19750789
(43) Veröffentlichungstag der Anmeldung: 15.11.2000
(73) Patentinhaber: Gesellschaft zur Beseitigung von Umweltschäden mbH, 04936 Schlieben (DE)
(72) Erfinder: FREIBERG, Jürgen, D-16431 Zepernick (DE); ZEHL, Gerald, D-12435 Berlin (DE); MEINKE, Martina, D-14193 Berlin (DE)
(74) Vertreter: Gulde, Klaus W., Dipl.-Chem.
(86) Internationale Anmeldenummer: EP9807102
(87) Internationale Veröffentlichungsnummer: WO99024163

(56) Entgegenhaltungen:
- EP-A- 0 737 661
- WO-A-96/17683
- GB-A- 2 234 450
- US-A- 4 499 205
- US-A- 5 426 253
- US-A- 5 663 438

## Beschreibung

Die Erfindung betrifft einen Tragerkatalysator und ein Verfahren zur Herstellung von Fluorkohlenwasserstoffen (FKW) durch Hydrodehalogenierung von Fluorchlorkohlenwasserstoffen (FCKW) oder Halonen mit Wasserstoff.

Zur Herstellung von Fluorkohlenwasserstoffen sind Verfahren vorgeschlagen worden, die die Umsetzung von Halogenkohlenwasserstoffen mit Fluorwasserstoff in Gegenwart von Friedel-Crafts-Katalysatoren (Gl. 1) und die Hydrodehalogenierung von chlor- oder bromhaltigen Fluorkohlenwasserstoffen mit Wasserstoff in Gegenwart eines Hydrierkatalysators (Gl. 2, 3) betreffen. Insbesondere der heterogenkatalytische Hydrodehalogenierungsprozeß ist kommerziell attraktiv. Beide Verfahrensprinzipien sind für die Produktion von Difluormethan CF₂H₂ in den Gleichungen 1 bzw. 2 und 3 beispielhaft dargestellt:

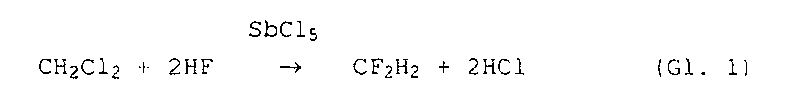

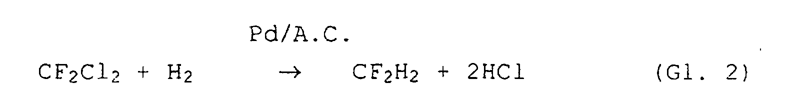

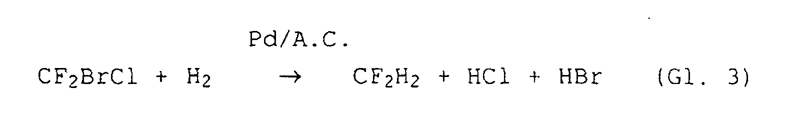

So entsteht nach EP-A-0669304 oder NL 9401574 A 960501 an Pd/C.-Katalysatoren durch Hydrodechlorierung von Dichlordifluormethan CF₂Cl₂ ein Rohdifluormethan CF₂H₂, welches durch nicht umgesetztes CF₂Cl₂ und weitere chlorhaltige Nebenprodukte verunreinigt ist. Ein Pt/A.C.-Katalysator nach JP 92-183134 920617 erlaubt gar nur die Umsetzung von CF₂Cl₂ mit einer Selektivität von 20% zum Zielprodukt CF₂H₂. Es werden 51% Chlordifluormethan gebildet. Ein nach JP 92-179322 920612 speziell präparierter PdCl₂/C-Katalysator ergab 61% CF₂H₂ und 23% Chlordifluormethan bei einem CF₂Cl₂-Umsatz von 80%.

Nach EP 508660 A1 921014 wurde Chlordifluormethan mit einer Selektivität von 74.3% zu CF₂H₂ umgesetzt. In WO 9617683 A1 960613 werden Palladium-Platin Hydrierkatalysatoren beschrieben, die die Umsetzung von Chlordifluormethan verbessern und bei vollständigem Umsatz von Chlordifluormethan bei Temperaturen von 350-400°C 91.3% CF₂H₂ liefern. Die beschriebenen Katalysatoren sind jedoch unter diesen drastischen Bedingungen nicht langzeitstabil. Weiterhin führen Beimischungen von Platin zu drastischen Selektivitätsverlusten beim Umsatz anderer technisch relevanter FCKW, wie CF₂Cl₂ oder Chlorpentafluorethan.

Pd/Al₂O₃-Katalysatoren, wie in EP-A-0742192 für die Umsetzung von Chlorpentafluorethan zu Pentafluorethan vorgeschlagen wurden, zeigen nicht die für eine technische Applikation erforderliche Langzeitstabilität.

Flurkohlenwasserstoffe erhalten nach Verboten und restriktivem Einsatz für Fluorchlorkohlenwasserstoffe als ozonschichtunschädliche Ersatzstoffe in der Kühlmittelund Treibgasapplikation steigende Bedeutung. Darüber hinaus ist die Entsorgung von FCKW und fluorhaltigen Halonen, die nach dem erfindungsgemäßen Verfahren als Rohstoffe eingesetzt werden, bisher noch nicht befriedigend gelöst.

Aufgabe der Erfindung ist die Schaffung eines leicht herstellbaren Trägerkatalysators für die FCKW- und Halonhydrodehalogenierung zu Fluorkohlenwasserstoffen, der eine im Vergleich zu anderen Katalysatoren höhere Aktivität bei hoher FKW-Selektivität aufweist und langzeitstabil ist, in Verbindung mit einem Hydrodehalogenierungsverfahren unter Einsatz dieses Katalysators.

Die Aufgabe wird erfindungsgemäß mit den kennzeichnenden Merkmalen des Anspruches 1 gelöst.

FCKW und fluorhaltige Halone werden hierbei in einer heterogenkatalytischen Gasphasenreaktion bei erhöhter Temperatur in Gegenwart von geträgerten Aktivkomponenten, Metallen der VIII. Nebengruppe, promotiert durch ein oder mehrere Elemente der III. und IV. Nebengruppe, mit hoher Aktivität und Selektivität langzeitstabil zu Fluorkohlenwasserstoffen hydrodehalogeniert.

Das erfindungsgemäße Verfahren ist dadurch gekennzeichnet, daß Verbindungen der allgemeinen Formel CH₃₋ _{(A+B+C)}F_{1+A}Cl_{B}Br_{c} (mit A+B+C≤3 und B+C≠0) in Gegenwart eines Trägerkatalysators, der auf einem von Verunreinigungen wie eisen- und zinkhaltigen Verbindungen freien Träger eine Aktivkomponentenmischung fixiert aufweist, die mindestens eine Metallzusammensetzung der allgemeinen Formel

PdₐX_{b}Y_{c}

enthält, in der
- X: Pt, Rh, Ir, Os und/oder Ru,
- Y: Ce, Hf, Y, Sc, Ti und/oder Zr,

- a: eine Zahl von 5 bis 100 - (b+c),
- b: eine Zahl von > 1 bis 100 - (a+c) und
- c: eine Zahl von > 0,5 bis 100 - (a+b)
bedeuten, wobei b+c≠0 sein muß,
umgesetzt werden.

Vorteilhafte Ausbildungen der Erfindung sind in den Unteransprüchen angegeben.

Als Katalysatorträger kommen Aktivkohle und anorganische Fluoride, Oxide und fluorierte Oxide, wie TiF₄, ZrF₃, AlF₃, Zr(OH)ₓF₃₋ₓ, Al₂O₃, TiO₂, ZrO₂ in Frage. Aus den Oxiden bilden sich während der katalytischen Reaktion fluoridhaltige Oxide. Bevorzugt wird Aktivkohle verwendet.

Die BET-Oberflächen liegen bei 10-1500 m²/g, vorzugsweise bei 50-1000 m²/g. Die Träger sollen frei von Verunreinigungen durch metallische Verbindungen, z.B. Verbindung des Eisens und Zinks sein. Dies kann z. B. bei Aktivkohle und säureunlöslichen Oxiden durch Wäsche mit konzentrierter Salzsäure bei erhöhter Temperatur erreicht werden.

Die Beladung des Trägers mit den Aktivkomponenten kann für Palladium 0.1 bis 40 Gew.%, die Metalle der VIII. Nebengruppe von 0 bis 30% allein oder als Mischung und für die Elemente der III. und IV. Nebengruppe, die sowohl als Träger als auch als Promotoren dienen können, von 0-99,9 Gew.% betragen.

Für die Präparation der Träger mit der Aktivkomponentenmischung werden an sich bekannte Methoden angewendet. Eine typische Herstellungsmethode des erfindungsgemäßen Katalysators erfolgt durch Imprägnation des Trägers mit löslichen Metallverbindungen bzw. hochfeinteiligen Dispersionen von Metallen (Metallsole) und Metallverbindungen oder über die Gasphase. So ist es möglich, eine wässrige Lösung der Metallsalze, z.B. der Chloride mit anschließender Trocknung des Katalysators, wie sie im Beispiel 1 beschrieben werden, aufzubringen. Die Imprägnation kann stufenweise in Form eines Schalenaufbaus mit einzelnen Bestandteilen der Aktivmetalle und Promotoren oder auch in einem Schritt erfolgen, indem eine Lösung mehrerer Aktivkomponentenverbindungen verwendet wird. Die Aktivkomponentenmischung, die auf dem Träger fixiert wird, enthält in Abhängigkeit von den einzelnen Ausgangsverbindungen entsprechende Liganden wie Säurereste und Komplexliganden,deren qualitative und quantitative Zusammensetzung sich unter den Reaktionsbedingungen verändern kann.

Der erfindungsgemäße Katalysator ist für die Hydrodechlorierung von halogenorganischen Verbindungen durch Umsetzung mit Wasserstoff bei erhöhter Temperatur einsetzbar. Die Ausgangsmaterialien sind FCKW, HFCKW und/oder Halone der allgemeinen Formel CH_{3-(A+B+C)}F_{1+A}Cl_{B}Br_{C} (mit B+C≠0 und A+B+C≤3). Die Konzentration der chlororganischen Verunreinigungen kann zwischen 0.001 bis 30 Vol.%, bevorzugt zwischen 0.01 und 10% betragen. Beispielsweise sind Ausgangsprodukte im Sinne des erfindungsgemäßen Verfahrens:
1. aus Kühlaggregaten zurückgewonnene Kältemittel, die chlorhaltige Bestandteile, wie Dichlordifluormethan, Chlordifluormethan und/oder Chlorpentafluorethan enthalten,
2. industriell hergestelltes Dichlordifluormethan und/oder Chlorpentafluorethan, daß durch eine selektive Hydrodechlorierung in Difluormethan oder Pentafluorethan gewandelt werden kann,
3. industriell hergestellte Fluorkohlenwasserstoffe (FKW), die durch chlorhaltige Neben- und/oder Zwischenprodukte verunreinigt sind.

Das erfindungsgemäße Verfahren wird in der Gasphase vorzugsweise bei Temperaturen von 130 - 400 °C und Drücken von 1 - 10 Mpa durchgeführt. Limitierend für den Druck sind Kondensation der im Edukt enthaltenen chlororganischen Verbindungen. Ein Edukt aus 48% Chlordifluormethan und 52% Chlorpentafluorethan kann bei einem Druck von 20 bar umgesetzt werden. Bevorzugt wird die Reaktion bei einem Druck von 1 bis 5 bar durchgeführt. Der Wasserstoffgehalt des Reaktionsgases beträgt dabei unterstöchiometrische bis mehrfach stöchiometrische Anteile, bezogen auf die Hydrodehalogenierung der chlororganischen Verbindungen. Vorteilhafterweise wird ein geringer stöchiometrischer Überschuß bezogen auf die selektive Hydrodechlorierung der chlororganischen Verbindungen eingesetzt. Überschüssiger, unreagierter Wasserstoff kann in den Prozeß zurückgeführt werden.

Die Verweilzeit der Reaktionsgase beträgt gewöhnlich 0.1 bis 80 sec, bevorzugt 2 - 20 sec. Sie ist abhängig von der Art und der Menge der umzusetzenden chlororganischen Verbindungen im Eduktgas.

Das erfindungsgemäße Verfahren zeichnet sich gegenüber dem Stand der Technik durch verbesserte Leistungen aufgrund insbesondere einer höheren Katalysatoraktivität und -stabilität des erfindungsgemäßen Trägerkatalysators aus.

Die Erfindung wird an Hand der folgenden Ausführungsbeispiele näher erklärt.

### Beispiel 1:

100g gebrochene und auf 1.0-1.2 mm Korngröße gesiebte Aktivkohle (z. B. MERCK) mit einer BET-Oberfläche von 1050 m²/g wird 4 Stunden bei 80°C in konzentrierter Salzsäure gerührt, über ein säurefestes Filter von der Waschlösung getrennt, zweimal mit konzentrierter Salzsäure gewaschen und in einem Rundkolben überführt. Die berechneten Mengen PdCl₂, OsCl₃ und ZrOCl₂ werden in 20%iger Salzsäure gelöst und zu der Aktivkohle addiert. Anschließend wird so viel destilliertes Wasser hinzugefügt, daß der Aktivkohleträger gerade mit Flüssigkeit bedeckt ist. Die Slurry wird dann am Rotationsverdampfer bei 50°C zur Trockne abgedampft.Der Katalysator erhält seine Einsatzform nach 10 Stunden Trocknen bei 120°C im Trockenschrank. Der Katalysator hat folgende Zusammensetzung (Gew. %) : 10 Teile Pd, 1 Teil Os, 0.5 Teile Zr, 87.5 Teile Aktivkohle ohne Berücksichtigung von Liganden. Die Präparation der in den Tabellen aufgeführten Katalysatormuster erfolgt nach der vorbeschriebenen gleichen Methode durch Variation der als Chloride oder Oxychloride verwendeten Metalle.

### Beispiel 2:

322.3 g ZrOCl₂ 8H₂O werden in 250 ml CH₂Cl₂ aufgeschlämmt und mit einer Lösung von 14.6 g Pd(ac)₂ und 5,1 g Iridiumacetylacetonat in 100 ml CH₂Cl₂ unter Rühren versetzt. Nach 1stündigem Rühren wird das Lösungsmittel am Rotationsverdampfer abgetrieben und der Rückstand 2h bei 110°C getrocknet. Der Katalysator hat folgende Zusammensetzung der Aktiv/Träger-Metalle (Gew.%) 91 Teile Zr, 7 Teile Pd und 2 Teile Iridium. Die trokkene krustige Masse wird gebrochen und auf 0.8-1.5 mm Korngröße ausgesiebt. Der Katalysator erhält seine aktive Form im Verlaufe einer Formierungsphase im Reaktor, in der während der Hydrierreaktion entstandener Fluorwasserstoff zur Bildung fluor- und chlorhaltiger Zirkonverbindungen führt.

### Beispiel 3:

45 cm³ (25.04 g) des Katalysators aus Beispiel 1 oder Beispiel 2 (1.0-1.2 mm Körnung) werden in ein elektrisch über eine LiNO₃/KNO₃-Salzschmelze beheiztes Reaktionsrohr aus V2A-Stahl (40 cm lang, 1.8 cm Durchmesser) gefüllt und im Argonstrom von 5 l/h auf 200°C geheizt. Nach 3 Stunden wird der Argonstrom durch das Reaktionsgas (15 l/h) ersetzt. Die Reaktorabgase passieren eine mit 20%iger Kalilauge gefüllte Waschflasche zur Absorption der sauren Gasbestandteile und ein mit Natronkalkgranulat versehenes Trockenrohr, bevor sie über eine Probenschleife der gaschromatischen Analyse unterzogen werden. Die Katalysatoreigenschaften werden im Temperaturbereich von 180-250°C untersucht.

### Beispiel 4:

Fünf Katalysatoren wurden nach den Präparationsmethoden der Beispiele 1 und 2 hergestellt und bezüglich ihrer Leistungsparameter, Aktivität und Selektivität bei der Umsetzung chlororganischer Verbindungen bei unterschiedlichen Temperaturen und unter Druck getestet. Beim Vergleich wurde ein Hydrodechlorierungskatalysator nach WO 96/17683 A1 940526 mit 10% Pd und 2% Pt auf Aktivkohle präpariert und in das Testprogramm integriert. In Tabelle 1 sind die Ergebnisse zusammengefaßt, die zeigen, daß unter Einsatz der beanspruchten metallmodifizierten Palladium-Trägerkatalysatoren eine sehr selektive und vollständige Umsetzung der FCKW in die entsprechenden FKW ermöglicht wird. Nichtmodifizierte Pd-Aktivkohle-Hydrodechlorierungskatalysatoren führen nicht zum gewünschten Erfolg.

**Tabelle 1:**

| Katalysatoren und deren katalytische Eigenschaften bei der Hydrodechlorierung von CF₂Cl₂ zu CH₂F₂ | | | | | | | |
|---|---|---|---|---|---|---|---|
| Nr. | Katalysator | Temperatur (°C) | Umsatz (%) | Selektivität (%) | | | |
| | | | | R32 | R22 | R41 | CH₄ |
| 1 | 10% Pd, 1% Os, 05 % Zr/AC | 220 | 96,4 | 78,2 | 1,8 | 0,1 | 19,9 |
| 2 | 91% Zr, 7% Pd, 2% Ir | 235 | 92,3 | 80,4 | 1,8 | 0,1 | 17,7 |
| 3 | 5% Pd, 5% Y, 1% Pt/AC | 230 | 97,2 | 83,4 | 1,1 | 0,4 | 15,1 |
| 4 | 5% Pd, 5% Ce, 1% Ru/AC | 230 | 96,9 | 83,1 | 1,4 | 0,2 | 15,3 |
| 5 | 5% Pd, 3% Ce, 2% Rh/AC | 230 | 97,1 | 82,1 | 1,2 | 0,1 | 16,6 |
| V | 10% Pd, 2% Pt/AC | 220 | 70,1 | 60,2 | 7,3 | 5,7 | 26,8 |

## Patentansprüche

1. Verfahren zur Herstellung von Fluorkohlenwasserstoffen durch Hydrodehalogenierung von Fluorchlorkohlenwasserstoffen (FCKW) und fluorhaltigen Halonen,
**dadurch gekennzeichnet,**
**daß** Verbindungen der allgemeinen Formel CH₃₋ _{(A+B+C)}F_{1+A}Cl_{B}Br_{c} (mit A+B+C≤3 und B+C≠0) in Gegenwart eines Trägerkatalysators, der auf einem von Verunreinigungen wie eisen- und zinkhaltigen Verbindungen freien Träger eine Aktivkomponentenmischung fixiert aufweist, die mindestens eine Metallzusammensetzung der allgemeinen Formel
PdₐX_{b}Y_{c}
enthält, in der
X Pt, Rh, Ir, Os und/oder Ru,
Y Ce, Hf, Y, Sc, Ti und/oder Zr,
a eine Zahl von 5 bis 100 - (b+c),
b eine Zahl von > 1 bis 100 - (a+c) und
c eine Zahl von > 0,5 bis 100 - (a+b)
bedeuten, wobei b+c≠0 sein muß,
umgesetzt werden.

2. Verfahren nach Anspruch 1,
**dadurch gekennzeichnet,**
**daß** im eingesetzten Trägerkatalysator für X und Y je ein oder mehrere Metalle stehen.

3. Verfahren nach Anspruch 1 oder 2,
**dadurch gekennzeichnet,**
**daß** als Träger Aktivkohle oder ein anorganisches Material auf der Basis von Metalloxiden, - fluoriden oder fluorierten Metalloxiden eingesetzt wird.

4. Verfahren nach einem der Ansprüche 1 bis 3,
**dadurch gekennzeichnet,**
**daß** eins Trägerkatalysator eingesetzt wird, der insgesamt 0.1 bis 50 Gewichtsprozent, vorzugsweise 0,5 - 20 Gewichtsprozent Aktivkomponenten enthält.

5. Verfahren nach einem der Ansprüche 1 bis 4,
**dadurch gekennzeichnet,**
**daß** ein Trägerkatalysator eingesetzt wird, dessen Aktivkomponentenmischung Liganden wie Säurereste und/oder Komplexliganden enthält.

6. Verfahren nach einem der Ansprüche 1 bis 5,
**dadurch gekennzeichnet,**
**daß** die Hydrodehalogenierung in Gegenwart von Wasserstoff in der Gasphase durchgeführt wird.

7. Verfahren nach einem der Ansprüche 1 bis 6,
**dadurch gekennzeichnet,**
**daß** Dichlordifluormethan (R12) und/oder Chlordifluormethan (R22) und/oder Chlorbromdifluormethan (R1211) zu Difluormethan (R32) umgesetzt werden.

8. Verfahren nach einem der Ansprüche 1 bis 7,
**dadurch gekennzeichnet,**
**daß** Chlorkohlenwasserstoffe, die als Verunreinigungen in Roh-Fluorkohlenwasserstoffen enthalten sind, hydrodehalogeniert werden.

9. Verfahren nach einem der Ansprüche 1 bis 8,
**dadurch gekennzeichnet,**
**daß** die Hydrodehalogenierung in der Gasphase bei Temperaturen von 130 - 400 °C durchgeführt wird.

10. Verfahren nach einem der Ansprüche 1 bis 9,
**dadurch gekennzeichnet,**
**daß** die Hydrodehalogenierung in der Gasphase bei 150°C bis 250°C durchgeführt wird.

11. Verfahren nach einem der Ansprüche 1 bis 10,
**dadurch gekennzeichnet,**
**daß** der Wasserstoff in unterstöchiometrischen bis mehrfach stöchiometrischen Anteilen bezogen auf die FCKW- und/oder Halonhydrodehalogenierung zu den Fluorkohlenwasserstoffen, eingesetzt wird.

12. Verfahren nach einem der Ansprüche 1 bis 11,
**dadurch gekennzeichnet,**
**daß** der Wasserstoff in stöchiometrischen bis 3-fach stöchiometrischen Anteilen, bezogen auf die FCKW- und/oder Halonhydrodehalogenierung zu den Fluorkohlenwasserstoffen, eingesetzt wird.

13. Verfahren nach einem der Ansprüche 1 bis 12,
**dadurch gekennzeichnet,**
**daß** die Hydrodehalogenierung im Kreislauf geführt wird, wobei Reaktionsprodukte teilweise und/oder vollständig vor Wiedereintritt in den Reaktionsbereich abgetrennt werden.

14. Verfahren nach einem der Ansprüche 1 bis 13,
**dadurch gekennzeichnet,**
**daß** der Gasphase inerte Gase zugesetzt werden.

## Claims

1. A process for the production of fluorohydrocarbons by hydrodehalogenation of fluorochlorocarbons (CFC) and fluorinated halons, **characterized in that** compounds of general formula CH_{3-(A+B+C)}F_{1+A}Cl_{B}Br_{C} (with A+B+C≤3 and B+C≠0) in the presence of a supported catalyst, having a mixture of active components fixed on a support which is free of contaminations such as compounds containing iron and zinc, which contains at least one metal composition of general formula
PdₐX_{b}Y_{c}
wherein
X represents Pt, Rh, Ir, Os and/or Ru,
Y represents Ce, Hf, Y, Sc, Ti and/or Zr,
a represents a number of from 5 to 100 - (b+c),
b represents a number of from >1 to 100 - (a+c) and
c represents a number of from >0,5 to 100 - (a+b);
with the requirement that b + c ≠ 0,
are reacted.

2. The process according to claim 1, **characterized in that** in the used supported catalyst each X and Y represents one or more metals.

3. The process according to claim 1 or 2, **characterized in that** active charcoal or an inorganic material on the basis of metal oxides, fluorides, or fluorinated metal oxides is used as support.

4. The process according to one of claims 1 to 3, **characterized in that** a support catalyst is used which contains from 0.1 to 50 wt.-%, preferably from 0.5 to 20 wt.-% of active components in total.

5. The process according to one of claims 1 to 4, **characterized in that** a support catalyst is used the mixture of active components of which contains ligands such as acid residues and/or complex ligands.

6. The process according to one of claims 1 to 5, **characterized in that** hydrodehalogenation is carried out in the gas phase in the presence of a hydrogen.

7. The process according to one of claims 1 to 6, **characterized in that** dichlorodifluoromethane (R12) and/or chlorodifluoromethane (R22) and/or chlorobromodifluoromethane (R1211) are reacted to form difluoromethane (R32).

8. The process according to one of claims 1 to 7, **characterized in that** chlorohydrocarbons contained as impurities in crude fluorohydrocarbons are subjected to said hydrodehalogenation.

9. The process according to one of claims 1 to 8, **characterized in that** the hydrodehalogenation is carried out in the gas phase at temperatures of from 130 to 400°C.

10. The process according to one of claims 1 to 9, **characterized in that** the hydrodehalogenation is carried out in the gas phase at temperatures of from 150 to 250°C.

11. The process according to one of claims 1 to 10, **characterized in that** hydrogen is used in a range from sub-stoichiometric to multi-stoichiometric percentages in the CFC and/or halon hydrodehalogenation to form fluorohydrocarbons.

12. The process according to one of claims 1 to 11, **characterized in that** hydrogen is used in a range from stoichiometric to threefold stoichiometric percentages in the CFC and/or halon hydrogenation to form fluorohydrocarbons.

13. The process according to one of claims 1 to 12, **characterized in that** the hydrodehalogenation is conducted in circulation, the reaction products being removed partially and/or completely prior to being capable of re-entering the reaction zone.

14. The process according to one of claims 1 to 13, **characterized in that** inert gases are added to the gaseous phase.

## Revendications

1. Procédé de préparation d'hydrocarbures fluorés par hydrodéshalogénation d'hydrocarbures fluorochlorés (CFC) et de halons fluorés, **caractérisé en ce que** l'on fait réagir des composés de formule générale CH_{3-(A+B+C)}F_{1+A}Cl_{B}Br_{C} (où A + B + C ≤ 3 et B + C ≠ 0) en présence d'un catalyseur supporté comprenant un mélange de composants actifs fixé sur un support exempt d'impuretés tels que des composés contenant du fer et du zinc, lequel mélange contient au moins une composition métallique de formule générale
PdₐX_{b}Y_{c}
dans laquelle
X représente Pt, Rh, Ir, Os et/ou Ru,
Y représente Ce, Hf, Y, Sc, Ti et/ou Zr,
a représente un nombre de 5 à 100 - (b + c),
b représente un nombre > 1 à 100 - (a + c) et
c représente un nombre > 0,5 à 100 - (a + b),
où b + c doit être ≠ 0.

2. Procédé selon la revendication 1, **caractérisé en ce que** dans le catalyseur supporté utilisé, X et Y représentent chacun un ou plusieurs métaux.

3. Procédé selon la revendication 1 ou 2, **caractérisé en ce que** le support utilisé est le charbon actif ou une substance inorganique à base d'oxydes ou de fluorures métalliques ou d'oxydes métalliques fluorés.

4. Procédé selon l'une quelconque des revendications 1 à 3, **caractérisé en ce que** l'on utilise un catalyseur supporté contenant au total de 0,1 à 50 pour cent en poids, de préférence de 0,5 à 20 pour cent en poids de composants actifs.

5. Procédé selon l'une quelconque des revendications 1 à 4, **caractérisé en ce que** l'on utilise un catalyseur supporté dont le mélange de composants actifs contient des ligands tels que des radicaux acides et/ou des ligands complexes.

6. Procédé selon l'une quelconque des revendications 1 à 5, **caractérisé en ce que** l'hydrodéshalogénation est réalisée en présence d'hydrogène en phase gazeuse.

7. Procédé selon l'une quelconque des revendications 1 à 6, **caractérisé en ce que** le dichlorodifluorométhane (R12) et/ou le chlorodifluorométhane (R22) et/ou le chlorobromodifluorométhane (R1211) sont mis à réagir pour donner lieu au difluorométhane (R32).

8. Procédé selon l'une quelconque des revendications 1 à 7, **caractérisé en ce que** des hydrocarbures chlorés qui sont présents en tant qu'impuretés dans des hydrocarbures fluorés bruts sont hydrodéshalogénés.

9. Procédé selon l'une quelconque des revendications 1 à 8, **caractérisé en ce que** l'hydrodéshalogénation en phase gazeuse est réalisée à des températures de 130 à 400°C.

10. Procédé selon l'une quelconque des revendications 1 à 9, **caractérisé en ce que** l'hydrodéshalogénation en phase gazeuse est réalisée entre 150°C et 250°C.

11. Procédé selon l'une quelconque des revendications 1 à 10, **caractérisé en ce que** l'hydrogène est utilisé en proportions sous-stoechiométriques jusqu'à plusieurs fois la stoechiométrie, par rapport à l'hydrodéshalogénation de CFC et/ou de halons pour donner lieu aux hydrocarbures fluorés.

12. Procédé selon l'une quelconque des revendications 1 à 11, **caractérisé en ce que** l'hydrogène est utilisé en proportions stoechiométriques jusqu'à 3 fois la stoechiométrie, par rapport à l'hydrodéshalogénation de CFC et/ou de halons pour donner lieu aux hydrocarbures fluorés.

13. Procédé selon l'une quelconque des revendications 1 à 12, **caractérisé en ce que** l'hydrodéshalogénation est répétée en un cycle, les produits de réaction étant séparés partiellement et/ou entièrement avant la ré-entrée dans le domaine de réaction.

14. Procédé selon l'une quelconque des revendications 1 à 13, **caractérisé en ce que** des gaz inertes sont ajoutés à la phase gazeuse.
